# EUROPÄISCHE PATENTSCHRIFT

(11) **EP 1 307 577 B1**
(45) Veröffentlichungstag und Bekanntmachung des Hinweises auf die Patenterteilung: **11.10.2006**
(21) Anmeldenummer: 01960525.2
(22) Anmeldetag: 20.07.2001
(51) Int. Cl.: C12P 7/42, C12R 1/645, C12R 1/785

(54) **VERFAHREN ZUR ENANTIOSELEKTIVEN REDUKTION VON 8-CHLOR-6-OXO-OCTANSÄUREALKYLESTERN**
METHOD FOR THE ENANTIOSELECTIVE REDUCTION OF 8-CHLORO-6-OXO-OCTANOIC ACID ALKYL ESTERS
PROCEDE DE REDUCTION ENANTIOSELECTIVE D'ALKYL ESTERS D'ACIDE 8-CHLORO-6-OXO-OCTANOIQUE

(30) Priorität: 27.07.2000 DE 10036515; 11.11.2000 DE 10056025
(43) Veröffentlichungstag der Anmeldung: 07.05.2003
(73) Patentinhaber: MEDA Pharma GmbH & Co. KG, 61352 Bad Homburg (DE)
(72) Erfinder: OLBRICH, Matthias, 01468 Reichenberg (DE); GEWALD, Rainer, 01217 Dresden (DE)
(86) Internationale Anmeldenummer: PCT/EP2001/008421
(87) Internationale Veröffentlichungsnummer: WO 2002/010422

(56) Entgegenhaltungen:
- EP-A- 0 939 132
- NAKAMURA K ET AL: "ASYMMETRIC REDUCTION OF KETONES BY GLYCEROL DEHYDROGENASE FROM GEOTRICUM" TETRAHEDRON LETTERS, ELSEVIER SCIENCE PUBLISHERS, AMSTERDAM, NL, Bd. 29, Nr. 20, 1988, Seiten 2453-2454, XP001021318 ISSN: 0040-4039 in der Anmeldung erwähnt
- DATABASE CA [Online] CHEMICAL ABSTRACTS SERVICE, COLUMBUS, OHIO, US; PATEL, RAMESH N. ET AL: "Stereoselective reduction of.beta.-keto esters by Geotrichum candidum" retrieved from STN Database accession no. 117:190226 XP002180887 & ENZYME MICROB. TECHNOL. (1992), 14(9), 731-8 ,
- SERVI S: "BAKER'S YEAST AS A REAGENT IN ORGANIC SYNTHESIS" SYNTHESIS, GEORG THIEME VERLAG. STUTTGART, DE, Bd. 93, 1990, Seiten 1-25, XP001018969 ISSN: 0039-7881 in der Anmeldung erwähnt

## Beschreibung

### Anwendungsgebiet der Erfindung

Die vorliegende Erfindung betrifft ein neues biokatalytisches Verfahren zur enantioselektiven Reduktion von prochiralen 8-Chlor-6-oxo-octansäurealkylestern der Formel I wahlweise mit Hilfe der Stämme Mucor racemosus bzw. Geotrichum candidum zum entsprechenden (S)- bzw. (R)-Enantiomer der Reaktionsprodukte. Nach Bildung des Chiralitätszentrums kann in bekannter Weise die weitere stereospezifische Umsetzung zu (R)-α-Liponsäure erfolgen (DE 195 33 881). Als Racemat wird α-Liponsäure vor allem zur Behandlung der diabetischen Neuropathie sowie akuter und chronischer Lebererkrankungen eingesetzt. Da ausschließlich das natürliche (R)-(+)-Enantiomer die biologische Aktivität aufweist, ist die asymmetrische Synthese dieses reinen Naturstoffes von großer Wichtigkeit.

### Charakteristik des bekannten Standes der Technik

Die Verbindungen 1 sind bekannt und dienen als Zwischenprodukte der großtechnischen Herstellung von racemischer Thioctsäure (M.W. Bullock et al., J. Am. Chem. Soc. 1954, 76, 1828).

Für die Herstellung der enantiomerenreinen (R)-α-Liponsäure werden sowohl chemische Syntheseverfahren, aber auch Verfahren mit biokatalytischen Teilstufen in der Literatur beschrieben (Übersichtsartikel: J.S. Yadav et al., J. Sci. Ind. Res. 1990, 49, 400). Chemische asymmetrische Syntheseverfahren benötigen in der Regel kostenintensive und komplizierte Ausgangsverbindungen, da z.B. die Möglichkeiten des Einsatzes der enantioselektiven Chemokatalyse an bestimmte elektronische und sterische Strukturmerkmale gebunden sind.

Bei den Herstellungsverfahren mit biokatalytischen Teilstufen werden zum einen Enzympräparationen von Lipasen und Oxydoreductasen und zum anderen Hefe eingesetzt.

Die bekannten lipasekatalysierten Verfahrensstufen (Y.R. Santosh Laxmi und D.S. lyengar, Synthesis, 1996, 594; N.W. Fadnavis und K. Koteshwar, Tetrahedron: Asymmetry 1997, 8, 337; N.W. Fadnavis et al., Tetrahedron: Asymmetry 1998, 9, 4109; S. Lee und Y. Ahn, J. Korean Chem. Sc. 1999, 43, 128) beruhen auf der Enantioselektivität der Esterspaltung zur Erzielung einer hohen Enantiomerenreinheit. Dabei werden racemische Gemische vorgelegt. Die biokatalytische Reaktion kann nur max. 50 % des racemischen Gemisches zur Gewinnung der enantiomerenreinen Verbindung nutzen. Das verbleibende unerwünschte Enantiomer muß entweder verworfen werden oder über aufwendige Reaktionsstufen zu einem racemischen Gemisch zurückverwandelt werden.
Verfahren mit Monooxygenasen (B. Adger et al., Bioorg. Biomed. Chem. 1997, 5, 253) benötigen kostenintensive Cofaktoren, wie NADH oder NADPH oder kostenintensive Cofactorrecyclingsysteme.

Für Hefe (Saccharomyces cerevisieae) sowie Pilze der Gattungen Mucor und Geotrichum ist bekannt, daß sie zur biokatalytischen Umsetzung von Intermediaten fähig sind.

Hefe wird bereits seit langer Zeit in Reduktionsreaktionen von β-Ketoestern, der Esterspaltung und anderen Synthesen als Biokatalysator eingesetzt (Übersichtsartikel: S. Servi , Synthesis 1990, 93,1).

Für Mucor-Arten (z. B. Mucor miehei und Mucor javanicus) sind insbesondere Reaktionen zur enantioselektiven Esterspaltung beschrieben. Während Mucor racemosus nur in Bezug auf die Reduktion von Tetramethylcyclohexandion (J. dÀngelo et al. J. Org. Chem. 1986, 51, 40) erwähnt wird, ist für Geotrichum candidum die biokatalytische Reduktion von β-Keto-Estern beschrieben (B. Wipf et al. Helv. Chim. Acta 1983, 66, 485).

Alle bisher bekannten Verfahren mit Hefe (A.S. Gopalan and H.K. Hollie, Tetrahedron Lett. 1989, 30, 5705; L. Dasaradhi et al., J. Chem. Soc., 1990, 729; M. Bezbarua et al., Synthesis, 1996, 1289; DE 40 37 440) bzw. Geotrichum candidum (B. Wipf et al. Helv. Chim. Acta 1983, 66, 485) vermögen nicht, Zwischenprodukte mit sauerstofffreien, kleinen Liganden in β-Position zum Reaktionszentrum enantioselektiv zu den entsprechenden S- bzw. R-Enantiomeren umzusetzen. Statt dessen werden über aufwendige Zwischenstufen große, sauerstoffhaltige Liganden in β-Position zur Keto-Gruppe eingeführt, die dann eine enantioselektive Umsetzung möglich machen.

In der hier beschriebenen Lösung wird gegenüber den bekannten Synthesen mit Ausgangsverbindungen gearbeitet, die nur sehr kleine Liganden in α- oder β-Stellung zur Ketogruppe tragen und trotzdem mit hoher Enantioselektivität umgesetzt werden.

Die hier dargestellte Lösung war gegenüber dem bekannten Stand der Technik besonders überraschend, da K. Nakamura et al. Tetrahedron Letters 29, 2453-4, 1988 beschreiben, dass nur Esterfunktionen und nicht Chloratome in Nachbarstellung zum Reaktionszentrum von Dehydrogenasen erkannt werden und zu enantioselektiven Umsetzungen führen.

### Darlegung des Wesens der Erfindung

Der Erfindung lag deshalb die Aufgabe zugrunde, unter Ausnutzung von bekannten Synthesebausteinen ein einfacheres und wirtschaftlicheres Verfahren für die asymmetrische Induktion innerhalb der (R)-α-Liponsäure-Synthesesequenz auf biokatalytischer Grundlage zu finden.

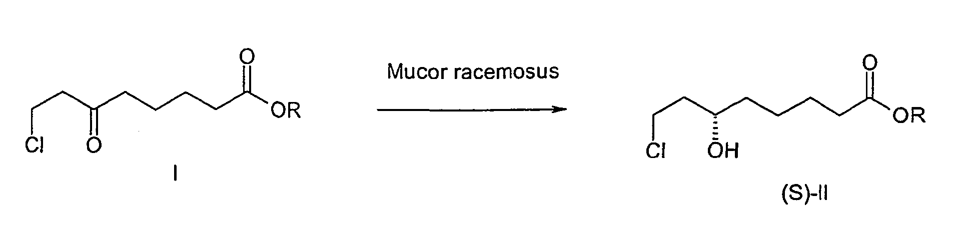

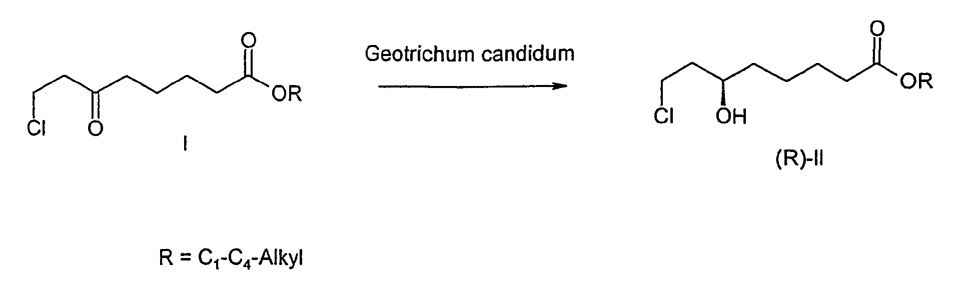

Erfindungsgemäß wurde die Aufgabe dadurch gelöst, dass neue Verfahren mit Mikroorganismen gefunden wurden, die eine enantioselektive Reduktion der prochiralen 8-Chlor-6-oxo-octansäurealkylester der Formel 1, worin R für C₁₋₄-Alkyl steht, wahlweise mit Hilfe der Stämme Mucor racemosus bzw. Geotrichum-candidum zum entsprechenden (S)- bzw. (R)-Enantiomer erlauben.
Als besonders geeignet hat sich 8-Chlor-6-oxo-octansäuremethylester erwiesen.

Die enantioselektive Reduktion prochiraler Verbindungen zum (R)-Enantiomer der Formel (R)-II erfolgt mit Hilfe von Geotrichum candidum. Eine Umsetzung der prochiralen Vorstufen zum (S)-Enantiomer der Formel (S)-II kann mittels Mucor racemosus erreicht werden.

Die hohe Enantioselektivität der Reduktion von 8-Chlor-6-oxo-octansäurealkylestern der Formel I war insofern nicht zu erwarten, da eine hohe asymmetrische Induktion in der Literatur nur für Verbindungen beschrieben wird, bei denen sterisch oder elektronisch stark unterschiedliche Gruppen auf beiden Seiten in α- oder β-Position zur Keto-Gruppe die Selektivität begünstigen. Desweiteren war überraschend, dass darüber hinaus zwei Stämme zu finden sind, die mit hoher Ausbeute und Enantioselektivität die Umsetzung zu entgegengesetzten Enantiomeren katalysieren.

Das erfindungsgemäße Verfahren zeichnet sich gegenüber dem Stand der Technik dadurch aus, daß zur Gewinnung der 8-Chlor-6-hydroxy-octansäurealkylester der Formel (R)-II Geotrichum candidum (DSM 13776) auf einem herkömmlichen Pilznähragar, vorzugsweise Sabouraud-Glukose-Agar, angezogen wird. Für die Vorkultur des Stammes wird ein komplexes Medium, vorzugsweise mit 1 % Hefeextrakt, 2 % Pepton und 2 % Glukose, verwendet. Eine weitere Vermehrung der Biomasse wird in einem vollsynthetischen Medium mit Glukose als Kohlenstoffquelle, Ammoniumsulfat als Stickstoffquelle sowie weiteren Nährsalzen erreicht, vorzugsweise in der Zusammensetzung 20 g/l Glukose, 3 g/l (NH₄)₂SO₄, 4 g/l KH₂PO₄, 0,5 g/l MgSO₄, 0,2 g/l NaCl, 0,2 g/l Hefeextrakt, 3 mg/l FeCl₃ x 6 H₂O, 3 mg/l CaCl₂ x 2 H₂O, 0,4 mg/l MnSO₄ x H₂O, 0,5 mg/l ZnSO₄ x 7 H₂O und 0,05 mg CuSO₄ x 5 H₂O. Die Kultivierung der Vorkultur als auch der Hauptkultur wird bei 19 bis 28 °C, vorzugsweise 24 °C, und über 1 bis 5 Tage, vorzugsweise 3 Tage, geschüttelt auf einem Rundschüttler bei 100 bis 300 rpm, vorzugsweise 190 rpm durchgeführt.

Die eigentliche biokatalytische Umsetzung wird in einer gepufferten wässrigen Lösung unter Zusatz von Glukose als Energiequelle durchgeführt. Die Konzentration des Biokatalysators beträgt 0,1 bis 100 g Biotrockenmasse pro Liter, vorzugsweise 5 g Biotrockenmasse pro Liter. Das Substrat wird in einer Konzentration von 5 g/l dem Biotransformationsansatz zugegeben. Die Biotransformation wird unter Schütteln bei 24 °C über 1 bis 3 Tage durchgeführt.
Nach Abschluß der Biotransformation wird die Biomasse abzentrifugiert und der Überstand mit einem organischen Lösungsmittel, vorzugsweise Ethylacetat, zweimal extrahiert. Der erhaltene Extrakt wird bis zur Trockene eingeengt. Das Rohprodukt enthält Anteile von (R)-8-Chlor-6-hydroxy-octansäure der Formel (R)-II (R=H), die auf bekannte Weise durch anschließende Veresterung mit in den jeweiligen Alkylester überführt werden (DE 195 33 881).
Die Kultivierung von Mucor racemosus (DSM 13775) sowie die Umsetzungen damit erfolgen analog wie für Geotrichum candidum beschrieben.
Das Rohprodukt enthält Anteile von (S)-8-Chlor-6-hydroxy-octansäure der Formel (S)-II (R=H), die auf bekannte Weise durch anschließende Veresterung in den jeweiligen Alkylester überführt werden (DE 195 33 881).
Die nach dem erfindungsgemäßen Verfahren hergestellten Verbindungen (R)-II und (S)-II weisen in der Regel einen hohen Enantiomerenüberschuss auf, entsprechend einer optischen Ausbeute von 70-95%. Die Enantiomerenverhältnisse werden direkt durch chirale Gaschromatographie an optisch aktiven Säulen gemessen.

### Ausführungsbeispiele

### Beispiel 1

Der Stamm Mucor racemosus (DSM 13775) wird auf einem Sabouraud-Agar bei 24 °C angezogen. 100 ml YPD-Nährlösung (1 % Hefeextrakt, 2 % Pepton und 2 % Glukose) wird mit einer Impföse beimpft und für 3 Tage bei 24 °C auf einem Rundschüttler (190 rpm) inkubiert. 10 % dieser Vorkultur werden in 100 ml SMG-Medium (20 g/l Glukose, 3 g/l (NH₄)₂SO₄, 4 g/l KH₂PO₄ ,0,5 g/l MgSO₄, 0,2 g/l NaCl, 0,2 g/l Hefeextrakt, 3 mg/l FeCl₃ x 6 H₂O, 3 mg/l CaCl₂ x 2 H₂O, 0,4 mg/l MnSO₄ x H₂O, 0,5 mg/l ZnSO₄ x 7 H₂O und 0,05 mg CuSO₄ x 5 H₂O) überführt und weitere 3 Tage bei 24 °C kultiviert.

Die erhaltene Biomasse wird abzentrifugiert und in 100 ml gepufferte wäßrige Lösung (50 mmol Na-Phosphatpuffer, pH 6,5) mit 5 g/l Glukose überführt. 0,5 g 8-Chlor-6-oxo-octansäuremethylester werden in 2 ml Ethanol gelöst und dem Biotransformationsansatz zugesetzt. Nach 24 h wird die Biomasse abgetrennt und das Medium zweimal mit je 50 ml Ethylacetat ausgeschüttelt. Die Extrakte werden vereinigt und das Lösungsmittel am Rotationsverdampfer abgezogen. Der Rückstand wird mit 10 ml Methanol aufgenommen und nach Zusatz von 0,04 ml konz. HCl 1 Stunde am Rückfluß erhitzt. Danach wird das Lösungsmittel abdestilliert. Nach säulenchromatographischer Reinigung (Kieselgel, Ethylacetat/Hexan=3:1) des Rückstandes erhält man 0,33 g (66%) (S)-8-Chlor-6-hydroxy-octansäuremethylester mit einem Enantiomerenüberschuß von 92% (chirale GC).

### Beispiel 2

Der Stamm Geotrichum candidum (DSM 13776) wird auf einem Sabouraud-Agar bei 24 °C angezogen. 100 ml YPD-Nährlösung (1 % Hefeextrakt, 2 % Pepton und 2 % Glukose) wird mit einer Impföse beimpft und für 3 Tage bei 24 °C auf einem Rundschüttler (190 rpm) inkubiert. 10 % dieser Vorkultur werden in 100 ml SMG-Medium (20 g/l Glukose, 3 g/l (NH₄)₂SO₄, 4 g/l KH₂PO₄ ,0,5 g/l MgSO₄, 0,2 g/l NaCl, 0,2 g/l Hefeextrakt, 3 mg/l FeCl₃ x 6 H₂O, 3 mg/l CaCl₂ x 2 H₂O, 0,4 mg/l MnSO₄ x H₂O, 0,5 mg/l ZnSO₄ x 7 H₂O und 0,05 mg CuSO₄ x 5 H₂O) überführt und weitere 3 Tage bei 24 °C kultiviert.

Die erhaltene Biomasse wird abzentrifugiert und in 100 ml gepufferte wässrige Lösung (50 mmol Na-Phosphatpuffer, pH 6,5) mit 5 g/l Glukose überführt. 0,5 g 8-Chlor-6-oxo-octansäuremethylester werden in 2 ml Ethanol gelöst und dem Biotransformationsansatz zugesetzt. Nach 24 h wird die Biomasse abgetrennt und das Medium zweimal mit je 50 ml Ethylacetat ausgeschüttelt. Die Extrakte werden vereinigt und das Lösungsmittel am Rotationsverdampfer abgezogen. Der Rückstand wird mit 10 ml Methanol aufgenommen und nach Zusatz von 0,04 ml konz. HCl 1 Stunde am Rückfluss erhitzt. Danach wird das Lösungsmittel abdestilliert. Nach säulenchromatographischer Reinigung (Kieselgel, Ethylacetat/Hexan=3:1) des Rückstandes erhält man 0,31 g (62%) (R)-8-Chlor-6-hydroxy-octansäuremethylester mit einem Enantiomerenüberschuss von 88% (chirale GC).

## Patentansprüche

1. Verfahren zur Herstellung von (R)-8-Chlor-6-hydroxy-octansäurealkylestern der Formel (R)-II aus 8-Chlor-6-oxo-octansäurealkylestern der Formel I, 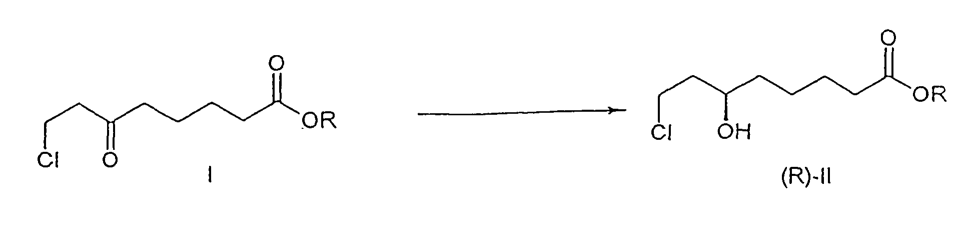 worin R jeweils die Bedeutung C₁₋₄-Alkyl hat, **dadurch gekennzeichnet, dass** man die Reaktion mittels eines Biokatalysators durchführt.

2. Verfahren zur Herstellung von (S)-8-Chlor-6-hydroxy-octansäuralkylestem der Formel (S)-II aus 8-Chlor-6-oxo-octansäurealkylestern der Formel I, 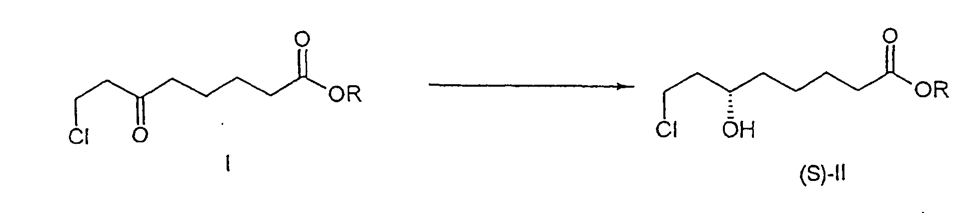 worin R jeweils die Bedeutung C₁₋₄-Alkyl hat, **dadurch gekennzeichnet, dass** man die Reaktion mittels eines Biokatalysators durchführt.

3. Verfahren nach den Ansprüchen 1 und 2, **dadurch gekennzeichnet, dass** man als prochirale Ausgangsverbindung 8-Chlor-6-oxo-octansäuremethylester einsetzt

4. Verfahren nach Anspruch 1, **dadurch gekennzeichnet, dass** man als Biokatalysator einen Stamm der Spezies Geotrichum candidum einsetzt.

5. Verfahren nach Ansprüchen 1 und 4, **dadurch gekennzeichnet, dass** man Geotrichum candidum mit dem speziellen Stamm (DSM 13776) einsetzt.

6. Verfahren nach Anspruch 2, **dadurch gekennzeichnet, dass** man als Biokatalysator einen Stamm der Spezies Mucor racemosus einsetzt.

7. Verfahren nach Ansprüchen 2 und 6, **dadurch gekennzeichnet, dass** man Mucor racemosus mit dem speziellen Stamm (DSM 13775) einsetzt.

8. Verfahren nach Ansprüchen 1, 2, 4 - 7, **dadurch gekennzeichnet, dass** man für die biotechnologische Produktion ein vollsynthetisches Medium benutzt, das eine Kohlenstoffquelle, eine anorganische Stickstoffquelle sowie weitere Nährsalze enthält.

9. Verfahren nach Ansprüchen 1 - 8, **dadurch gekennzeichnet, dass** im Verfahren anfallende (R)-8-Chlor-6-hydroxy-octansäure der Formel (R)-II oder (S)-8-Chlor-6-hydroxy-octansäure der Formel (S)-II, worin R jeweils die Bedeutung Wasserstoff hat, durch Veresterung in bekannter Weise zu dem Alkylester der Formel (R)-II oder (S)-II, worin R jeweils C₁₋₄-Alkyl bedeutet, umgesetzt wird.

## Claims

1. Process for the production of (R)-8-chloro-6-hydroxyoctanoic acid alkyl esters of the formula (R)-II from 8-chloro-6-oxo-octanoic acid alkyl esters of the formula I, 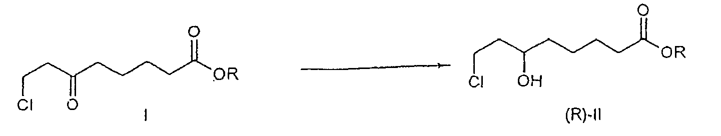 in which R in each case means C₁₋₄ alkyl, **characterised in that** the reaction is performed by means of a biocatalyst.

2. Process for the production of (S)-8-chloro-6-hydroxyoctanoic acid alkyl esters of the formula (S)-II from 8-chloro-6-oxo-octanoic acid alkyl esters of the formula I, 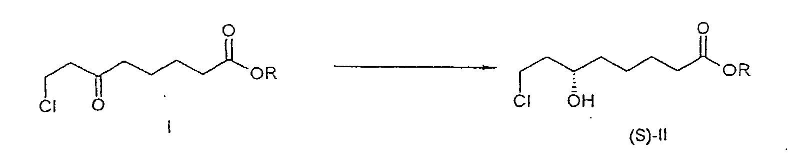 in which R in each case means C₁₋₄ alkyl, **characterised in that** the reaction is performed by means of a biocatalyst.

3. Process according to Claims 1 and 2, **characterised in that** 8-chloro-6-oxo-octanoic acid methyl ester is used as the prochiral starting compound.

4. Process according to Claim 1, **characterised in that** a strain of the species Geotrichum candidum is used as the biocatalyst.

5. Process according to Claims 1 and 4, **characterised in that** Geotrichum candidum of the specific strain (DSM 13776) is used.

6. Process according to Claim 2, **characterised in that** a strain of the species Mucor racemosus is used as the biocatalyst.

7. Process according to Claims 2 and 6, **characterised in that** Mucor racemosus of the specific strain (DSM 13775) is used.

8. Process according to Claims 1, 2, 4-7, **characterised in that** a completely synthetic medium is used for biotechnological production, which medium contains a carbon source, an inorganic nitrogen source together with further nutrient salts.

9. Process according to Claims 1-8, **characterised in that** any (R)-8-chloro-6-hydroxyoctanoic acid of the formula (R)-II or (S)-8-chloro-6-hydroxyoctanoic acid of the formula (S)-II, in which R in each case means hydrogen, arising in the process is converted by esterification in known manner into the alkyl ester of the formula (R)-II or (S)-II, in which R in each case means C₁₋₄ alkyl.

## Revendications

1. Procédé de préparation d'esters alkyliques de l'acide (R)-8-chloro-6-hydroxy-octanoïque de formule (R)-II à partir d'esters alkyliques de l'acide 8-chloro-6-oxo-octanoïque de formule I 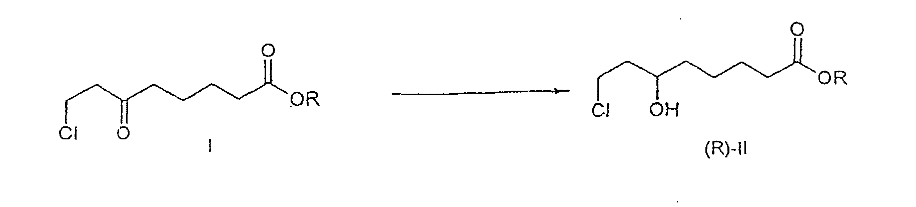 où R représente dans tous les cas un radical alkyle en C₁₋₄, **caractérisé en ce qu'**on met en oeuvre la réaction à l'aide d'un biocatalyseur.

2. Procédé de préparation d'esters alkyliques de l'acide (S)-8-chloro-6-hydroxy-octanoïque de formule (S)-II à partir d'esters alkyliques de l'acide 8-chloro-6-oxo-octanoïque de formule I 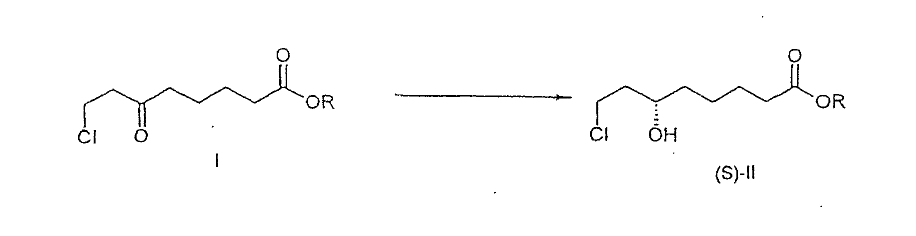 où R représente dans tous les cas un radical alkyle en C₁₋₄, **caractérisé en ce qu'**on met en oeuvre la réaction à l'aide d'un biocatalyseur.

3. Procédé selon les revendications 1 et 2, **caractérisé en ce qu'**on utilise en tant que composé de départ prochiral l'ester méthylique de l'acide 8-chloro-6-oxo-octanoïque.

4. Procédé selon la revendication 1, **caractérisé en ce qu'**on utilise en tant que biocatalyseur une souche de l'espèce Geotrichum candidum.

5. Procédé selon les revendications 1 et 4, **caractérisé en ce qu'**on utilise Geotrichum candidum avec la souche spéciale (DSM 13776).

6. Procédé selon la revendication 2, **caractérisé en ce qu'**on utilise en tant que biocatalyseur une souche de l'espèce Mucor racemosus.

7. Procédé selon les revendications 2 et 6, **caractérisé en ce qu'**on utilise Mucor racemosus avec la souche spéciale (DSM 13775).

8. Procédé selon les revendications 1, 2, 4-7, **caractérisé en ce qu'**on utilise pour la production biotechnologique un milieu entièrement synthétique qui contient une source de carbone, une source d'azote inorganique, ainsi que d'autres sels nutritifs.

9. Procédé selon les revendications 1-8, **caractérisé en ce qu'**on fait réagir l'acide (R)-8-chloro-6-hydroxy-octanoïque de formule (R)-II ou l'acide (S)-8-chloro-6-hydroxy-octanoïque de formule (S)-II obtenus dans le procédé, où R représente dans chaque cas un atome d'hydrogène, par estérification d'une manière connue, pour obtenir l'ester alkylique de formule (R)-II ou (S) -II, où R représente dans chaque cas un radical alkyle en C₁₋₄.
